Europäisches Patentamt

European Patent Office

Office européen des brevets

(19)

(11) Veröffentlichungsnummer: **0 100 451**
**B1**

(12) **EUROPÄISCHE PATENTSCHRIFT**

(45) Veröffentlichungstag der Patentschrift:
**05.11.86**

(21) Anmeldenummer: **83106481.1**

(22) Anmeldetag: **02.07.83**

(51) Int. Cl.⁴: **C 07 C 47/575,** C 07 C 45/64,
C 07 C 37/02

(54) Verfahren zur Hydroxylierung von aromatischen Verbindungen.

(30) Priorität: **02.08.82 US 404543**

(43) Veröffentlichungstag der Anmeldung:
**15.02.84 Patentblatt 84/7**

(45) Bekanntmachung des Hinweises auf die Patenterteilung:
**05.11.86 Patentblatt 86/45**

(84) Benannte Vertragsstaaten:
**DE FR IT**

(56) Entgegenhaltungen:
**US-A-3 855 306**

**CHEMICAL ABSTRACTS, Band 30, 1936, Columbus,
Ohio, USA, H. ERDTMAN "Dehydrogenation of
phenols", spalte 449
CANADIAN JOURNAL OF CHEMISTRY, Band 40,
Juli 1962, Ottawa, S.K. BANERJEE et al. "The
synthesis of lignin model substances:
5-hydroxyvanillin and 5-hydroxy-acetoguaiacone",
Seiten 2175-2177**

(73) Patentinhaber: **ITT INDUSTRIES INC., 320 Park
Avenue, New York, NY 10022 (US)**

(72) Erfinder: **Cooper, Geoffrey Kenneth, E.180
Ridgeview Dr., Shelton WA 98584 (US)**
Erfinder: **Harruff, Lewis Gregory, Star Rte.1 Box
215, Grapeview, WA 98546 (US)**

(74) Vertreter: **Morstadt, Volker, Dipl.- Ing., c/o
Deutsche ITT Industries GmbH
Patent/Lizenzabteilung Postfach 840 Hans- Bunte-
Strasse 19, D-7800 Freiburg/Brsg. (DE)**

## Beschreibung

Die Erfindung betrifft ein Verfahren zur Hydroxylierung aromatischer Verbindungen mit als Elektronendonatoren wirkenden Substituenten am Kern.

Vom Lignin abstammende aromatische Verbindungen sind häufig billige Vorstufen einer Anzahl wertvoller organischer Substanzen. So ist z.B. Vanillin eine wichtige Vorstufe von 3,4,5-Trimethoxybenzaldehyd, einer chemischen Substanz von hinlänglich bekannter Bedeutung für die pharmazeutische Industrie. Die bekannten Verfahren zur Umwandlung von Vanillin in Trimethoxybenzaldehyd haben jedoch bestimmte Nachteile. In dem US Patent 38 55 306 wird ein derartiges Verfahren beschrieben, bei dem Vanillin zur Herstellung von 5-Bromvanillin unter Verwendung von Brom und konzentrierter Bromwasserstoffsäure als Lösungsmittel bromiert wird. Das resultierende 5-Bromvanillin wird aus dem Reaktionsgemisch isoliert und zum entsprechenden Hydroxyvanillin mit Natriumhydroxid und einem Kupferkatalysator hydrolisiert. Die resultierende Reaktionsmischung enthält Natriumbromid, das üblicherweise nicht in den Kreislauf zurückgeführt sondern eher verworfen oder anderweitig verwendet wird. Darüberhinaus sind Brom und Bromwasserstoffsäure beide hochkorrosiv und der Umgang mit ihnen ist nicht ungefährlich.

Andere halogenierte Vanillinderivate schließen 5-Chlor-vanillin und 5-Jodvanillin ein. Es ist jedoch bekannt, daß Chlorvanillin äußerst schwer mit OH-Gruppen bei Kupferkatalyse reagiert und man selbst unter Bedingungen, die gegenüber den bei der Umwandlung von 5-Bromvanillin in 5-Hydroxyvanillin angewandten wesentlich schärfer sind, kein Hydroxyvanillin erhält. Andererseits sind Aryliodide als wesentlich reaktiver als die entsprechenden Bromverbindungen bei dieser Reaktionsart bekannt. Jod jedoch wäre unerschhinglich teuer, wenn man es wie Brom bei der oben beschriebenen Bromierungsreaktion verwerfen würde. Bezüglich der Jodierung von Vanillin zu 5-Jodvanillin wird Bezug genommen auf H.Erdman, Svensk.Kem.Tids, 47, 223 (1935) Chemical Abstracts 30:449, bezüglich der Umwandlung von 5-Jodvanillin in 5-Hydroxyvanillin auf S.Banerjee, M.Manolopoulo und J.M.Pepper, Canadian Journal of Chemistry 40, 2175 (1962). Bei den beschriebenen Verfahren wird jedoch die Zwischenstufe Jodvanillin vor der Hydrolyse isoliert und das anfallende Iodidion wird aus den verschiedenen Reaktionen nicht zurückgewonnen.

Hier will die Erfindung Abhilfe schaffen. Die Erfindung, wie sie in den Ansprüchen gekennzeichnet ist, löst die Aufgabe, ein Verfahren anzugeben, das wesentlich wirtschaftlicher arbeitet als die bislang bekannt gewordenen.

Das erfindungsgemäße Verfahren offenbart die Umwandlung einer aromatischen Verbindung in die entsprechende Hydroxylverbindung ohne Isolierung der als Zwischenstufe auftretenden halogenierten Verbindung und erlaubt die wirtschaftliche Rückgewinnung und Wiederverwendung der Halogennebenprodukte, die im Verlauf der Reaktion gebildet werden. Das Verfahren gemäß den Patentansprüchen umfaßt die Hydroxylierung einer aromatischen Verbindung durch Reaktion derselben in Gegenwart von Wasser als Lösungmittel mit einem Triiodidreagenz unter Ausbildung eines Reaktionsgemisches, daß die entsprechende jodaromatische Verbindung enthält, Umsetzen der Mischung ohne Abtrennen der jodaromatischen Verbindung mit einem hydroxylierenden Mittel, wodurch die entsprechende Hydroxylverbindun und zusätzlich Jodidsalz gebildet wird, Trennen der hydroxyaromatischen Verbindung von dem Jodidsalz und Zurückverwandlung des Jodidsalzes in Jod. Die entsprechende alkoxyaromatische Verbindung kann durch Alkylierung der Hydroxylverbindung nach einem der bekannten Alkylierungsverfahren hergestellt werden.

Das erfindungsgemäße Verfahren macht bei Anwendung des Jodierungsweges aus einem unwirtschaftlichen ein wirtschaftliches Verfahren. Rei einem solchen Verfahren wie die Umwandlung von Vanillin zu Trimethoxibenzaldehyd wird das Verfahren zu einem Eintopf-Verfahren" dadurch, daß die Reinigung der Zwischenstufe 5-Jodvanillin wegfällt. Mit anderen Worten, Jodierung und Umwandeln des Jodvanillins zu Hydroxyvanillin kann in dem gleichen Reaktionsgefäß durchgeführt werden. Darüberhinaus erübrigt die effiziente Rückführung des Nebenproduktes Iodidsalz in das bei der Reaktion verwendete Triiodidreagenz die Notwendigkeit, das wertvolle Jod/Iodidmaterial zu beseitigen. Bei Verfahren nach dem Stand der Technik wird notwendigerweise das Bromid- oder Iodidnebenprodukt aus der Halogenierungsreaktion und ebenso aus der Hydroxylierungsreaktion abgetrennt und zurückgewonnen. Beim vorliegenden Verfahren wird das Iodidsalz lediglich nach der Hydroxylierungsreaktion zurückgewonnen, zu einem Zeitpunkt, an dem es zu Jod oxidiert und teilweise zur Ausbildung des Triiodid enthaltenden Startmaterials reduziert werden kann. (Das Triiodidreagenz ist eine Lösung von Jod in einem Überschuß an Iodidsalz z.B. Natriumiodid plus Jod oder Natriumtriiodid). Wird Chlor als Oxidationsmittel verwendet, so ist das einzige reine Nebenprodukt der Reaktion Natriumchlorid, ein bekanntlich billiges Abfallprodukt.

Die sich zur Anwendung der vorliegenden Erfindung eignenden Startsubstanzen sind aromatische Verbindungen, die elektrophilen Substitutionsreaktionen zugänglich sind. Das Verfahren ist für aromatische Verbindungen geeignet, die als Elektronendonatoren wirkende Substituenten enthalten, wie z.B. mono- oder polyzyklische aromatische Verbindungen mit einem oder mehreren Kohlenwasserstoffresten wie z.B. Alkyl, Cycloalkyl, Aryl oder Arylalkylgruppen und/oder eine oder mehrere Hydroxygruppen oder Aldehyd-, Säure-, Esterreste wie z.B. Alkoxy-, Carboxy-, Carboxyl- oder Aldehyd-Carbonyl-Gruppen. Das Verfahren ist ungeeignet bei Substituenten wie Poly-Nitro- oder Ketegruppen mit einem Alpha-Wasserstoffatom, die entweder mit dem Reagenz reagieren oder den Ring stark deaktivieren. Ketogruppen sind wie Aldehydgruppen deaktivierend, sie enthalten jedoch ein Alpha-Wasserstoffatom, das mit dem Iodierungsmittel zur Reaktion gebracht werden kann, was bei Aldehyden nicht der Fall ist. Bei Diarylketonen gibt es z.B. keine Störung. Nur im Fall von stark deaktivierenden Gruppen wie z.B. Poly-Nitrogruppen ist die Deaktivierung ein Problem bei der Durchführung der Iodierung. Schwach deaktivierende Gruppen wie Aldehydgruppen beeinträchtigen die Iodierung nicht. Geeignete aromatische Verbindungen sind einfache einwertige Phenole wie Phenol selbst, Ortho-/Meta- und Para-Cresol und Guaiacol, mehrwertige Phenole wie Catechol und Resorcinol, phenolische Aldehyde wie Protocatechualdehyd, Vanillin, Syringaldehyd, p-Hydroxybenzaldehyd und 5-Formylvanillin, ferner phenolische Säuren wie Vanillinsäure, Syringasäure, Protocatechusäure und p-Hydroxybenzosäure. Die bevorzugten aromatischen Reaktanten sind solche mit mindestens einer phenolischen Hydroxylfunktion, insbesondere solche mit sowohl einer Aldehydgruppe als auch einem Hydroxyoder Alkoxysubstituenten.

Der erste Schritt der Reaktion besteht in der Iodierung der aromatischen Verbindung mit dem Triiodidreagenz in Anwesenheit von Wasser als Lösungsmittel. Das Wasser sollte 0,7 bis 1,25 Mol Äquivalente an Hydrox, vorzugsweise einem Alkalihydroxid, und 1 bis 2 Mol Äquivalente an Alkalitriiodid (z.B. Jod plus Natriumiodid) enthalten. Das wässrige Lösungsmittel sollte auch 0,1 bis 20 mol% eines sauren Katalysators, der Mineralsäure wie Schwefelsäure, Fluorwasserstoffsäure oder Phosphorsäure sein kann, enthalten. Die Reaktion wird bei einem Temperaturbereich von 20 bis 120°C ausgeführt. Die Ausgangsverbindung wird zum Substituenten am Kern jodiert in Ortho- oder Parastellung.

Der nachfolgende Reaktionsschritt, die Hydroxylierung, erfolgt direkt mit dem Reaktionsgemisch der Iodierung ohne Tsolierung der Zwischenstufe oder anderweitige Verarbeitung der Reaktanten oder Beiprodukte. Eine Base, wie z.B. Alkalihydroxid oder quarternäres Amin wie Tetraalkylammoniumhydroxid, wird zur Einstellung einer 0,5 bis 6 molaren Endkonzentration der Reaktionsmischung direkt zugefügt, zusammen mit 0,1 bis 20mol% an Kupfer oder einem Kupfersalz wie CuO, CuCl$_2$ oder CuJ$_2$, und zwar bei Temperaturen zwischen 50 und 120°C. Die bevorzugten Bedingungen sind Zufügung von Natriumhydroxid zum Iodierungsgemisch bis zu einer 2 bis 5 molaren Konzentration, dann Zusetzen von 1 bis 5 mol% Kupferstaub, Kupfer-(I)Oxid oder Kupfer(I)Chlorid, dann zum Rückfluß erhitzen des Reaktionsgemisches 18 Stunden bei 100 bis 120°C.

Das aus Natriumiodid oder einem anderen Iodid bestehende Nebenprodukt lässt sich durch Neutralisieren des Laugenanteils in dem Reäktionsgemisch mit Schwefel- oder Salzsäure rückgewinnen, indem man den organischen Anteil aus der wäßrigen Lösung extrahiert und dann diese mit einem Oxidationsmittel behandelt. Dieses kann Chlor, Natriumhypochlorit, Wasserstoffperoxid, Persulfat, Perborat sein, man aber auch eine elektrochemische Oxidation vornehmen. Das ausgefallene Jod wird aus der wässrigen Lösung abgefiltert, extrahiert oder destilliert/sublimiert. Die Temperatur der wässrigen Phase kann zwischen 0 und 100°C liegen. Das bevorzugte Verfahren zur Jodrückgewinnung ist die Behandlung der wässrigen Lösung mit Schwefelsäure zur Neutralisierung der Base, Extraktion des organischen Anteils mit einem Lösungsmittel wie Methylenchlorid oder Toluol, Oxidation mit Chlor oder auf elektrochemischem Wege und Filtration oder Lösungsmittelextratkon zur Rückgewinnung des Jods. Die rohe hydroxyaromatische Verbindung kann dann direkt einem beliebigen nachfolgenden Alkylierungsprozeß unterworfen werden.

Ein spezieller Verfahrensablauf der vorliegenden Erfindung mit Vanillin als aromatischer Verbindung lautet wie folgt: Vanillin wird in Wasser unter Erwärmen auf 50 bis 100°C gelöst, wobei die Lösung ein Moläquivalent Natriumhydroxid enthält. Ein Äquivalent Jod und zwei Äquivalente Natriumiodid werden der wässrigen Lösung zugefügt, so daß man ein Mol NaJ$_3$ - NaJ erhält. Die Natriumtriiodidlösung wird der Natriumvanillatlösung zusammen mit einer katalytischen Menge an Schwefelsäure, vorzugsweise 5 bis 10 mol%, zugesetzt. Die Mischung wird eine Stunde bei 50 bis 100°C gerührt, dann Natriumhydroxid zugefügt, um die Lösung alkalisch zu machen (von 1 auf 5n). Der Kupferkatalysator wird dann zugefügt und die Mischung solange zum Rückfluß erhitzt, bis das Jodvanillin verbraucht ist (nach ca. 12Std.). Der Überschuß an Hydroxid wird daraufhin neutralisiert und das 5-Hydroxyvanillin mit einem wasserunlöslichen organischen Lösungsmittel aufgenommen. Die wäßrige Phase mit dem Natriumiodid wird der Oxidation unterworfen und das resultierende Jod fällt aus der Lösung aus. Die festen Anteile werden abfiltriert, und eine Natriumtriiodidlösung durch Reduktion eines Teils des Jods zu Natriumiodid und Auflösen des Jods in dem Iodid hergestellt.

Die Alkylierung der hydroxyaromatischen Verbindung zu der entsprechenden alkoxyaromatischen Verbindung läßt sich gemäß den bekannten Alkylierungsverfahren derartiger Verbindungen durchführen, bei denen diese mit Alkylsulfat, Alkylhalogenid oder Alkylsulfonat in einem entsprechenden Lösungsmittel umgesetzt werden, wobei dieses vorzugsweise Wasser ist, das eine Base wie Natriumhydroxid enthält. Derartige Reaktionen lassen sich an vielen Stellen der Literatur nachlesen, z.B. in Organic Synthesis, Col. Vol. II, Seite 619, 1943, wo Veratral aus Vanillin hergestellt wird. Das Jodsalz kann ggf. im Anschluß an die Alkylierungsreaktion zurückgewonnen werden.

Die folgenden Beispiele erläutern die vorliegende Erfindung. Wenn nicht anders angegeben, sind Teile- und Prozentangaben auf das Gewicht bezogen.

## Beispiel 1

3,04g (0,02mol) Vanillin wurden in 20m1 1n-Natronlauge gelöst und auf 80°C erwärmt. Dann wurde innerhalb 30 Min. tropfenweise eine Lösung aus $NaJ_3$ - NaJ (2n) in 10,1ml Wasser plus 0,5ml 20%iger wäßriger $H_2SO_4$ zugefügt und die Mischung weitere 30 Minuten gerührt. 7,6ml einer 50%igen Natronlauge und Kupferstaub (128mg = 10mol%) wurden daraufhin zugefügt und die Mischung über Nacht zum Rückfluß erhitzt. Die Lösung wurde abgekühlt, durch Filtrieren vom Katalysator befreit, mit 20%iger wäßriger $H_2SO_4$ neutralisiert und gründlich mit Chloroform extrahiert. Nach aem Trocknen der organischen Phase über $Na_2SO_4$ und Abdampfen des Lösungsmittels verblieben 3,1g (99%) organisches Material, bestehend aus 75% 5-Hydroxy vanillin und 25% Vanillin.

Die wässrige Phase wurde im Vakuum eingeengt und mit der theoretischen Menge an Chlor behandelt. Die purpurfarbenen Jodkristalle wurden abfiltriert, 87% an Jod wurde zurückgewonnen.

## Beispiel 2

2,84g Vanillin wurden in 20ml n-NaOH bei 80°C gelöst und dann eine Mischung aus 2n $NaJ_3$ - NaJ/$H_2O$ (10,1ml) plus 20% wäßriger $H_2SO_4$ (0,5ml gleich 8mol%) tropfenweise innerhalb 30 Minuten zugefügt. Es bildete sich ein fahlgelber Niederschlag. Das Reaktionsgemisch wurde weitere 3,5 Std. gerührt. Um den Überschuß an Jod zu verringern, wurden zunächst 1,5ml einer 10%igen Lösung von $Na_2S_2O_3$ zugefügt, dann 7,6ml 50%ige wässrige Natronlauge zugesetzt, (um den Gehalt an NaOH auf 4n zu bringen), und schließlich 128mg Kupferstaub zugesetzt. Die Mischung wurde über Nacht zum Rückfluß erhitzt, auf Raumtemperatur abgekühlt, der Katalysator abfiltriert und der pH-Wert mit 20%iger wäßriger Schwefelsäure auf pH2 gestellt. Die Lösung wurde 5 mal mit einem Gemisch aus Methanol/Chloroform (20:80) extrahiert, es resultierten 3,1g (99%) eines Produkts, das nach dem NMR-Spektrum zu 75% aus 5-Hydroxi-vanillin und 25% Vanillin bestand.

Beispiel 2 wurde unter Verwendung von Natronlaugemengen in der Höhe 1 n bis 6 n, unter Verwendung von KOH und LiOH anstelle von NaOH beim Iodierungsprozeß wiederholt. Es resultierten im wesentlichen die gleichen Ergebnisse.

Beispiel 2 wurde ebenfalls unter der Verwendung von 5 bis 10 mol% Kupfer-I-Oxid, Kupfer-I-Chlorid, Kupfer-I-Iodid und Kupferstaub als Katalysator wiederholt. Die Ausbeute an 5-Hydroxgvanillin betrug 80 bis 85% mit Kupferstaub, 70 bis 80% (bezogen jeweils auf Vanillin) mit den anderen Katalysatoren.

## Beispiel 3

3,04g Vanillin wurden Iodiert und in 5-Hydroxyvanillin entsprechend den Beispielen 1 umgewandelt. Die wäßrige Phase der Extraktion von 5-Hydroxyvanillin (theoretischer Gehalt an Natriumiodid 0,8mol) wurde im Vakuum eingeengt, um flüchtige gelöste organische Anteile zu entfernen, und daraufhin Chlorwasser langsam zugesetzt (0,04mol $CO_2$ = 500ml 0,068 mChlorwasser) Der Jodniederschlag wurde abfiltriert und mit Wasser gewaschen. Zur Bestimmung der rückgewonnenen Menge wurde das Jod mit 2 n Natriumiodidlösung (300ml) vom Filter gewaschen, bei pH 5 mit 0,2 normaler Natriumthiosulfatlösung titriert.

Der Verbrauch an 0,2 normaler Thiosulfatlösung lag bei 350ml was 0,07mol rückgewonnenem Jod entspricht und einen Wirkungsgrad von 87% anzeigt.

Das erfindungsgemäße Verfahren ist also im wesentlichen ein Eintopfverfahren zur weitgehenden Umwandlung von aromatischen Verbindungen in die entsprechenden hydroxyaromatischen Verbindungen und zur Rückgewinung und Rückfürung der bei der Umwandliung verwendeten Jodidreagenzien.

## Patentansprüche

1. Verfahren zur Hydroxylierung einer aromatischen Verbindung mit mindestens einem als Elektronendonator wirkenden Substituenten am Kern, wobei ein an den Kern gebundenes Jodatom gegen eine Hydroxylgruppe ausgetauscht wird, dadurch gekennzeichnet,
- daß die aromatische Verbindung in Wasser mit einem Triiodidreagenz zu einem Reaktionsgemisch aus jodaromatischer Verbindung und Iodidsalz umgesetzt wird,
- daß dem Reaktionsgemisch ein hydroxylierendes Mittel zugesetzt und das Jodatom der jodaromatischen Verbindung gegen die Hydroxylgruppe ausgetauscht wird unter Entstehung von weiterem Iodidsalz, und

- daß die hydroxyaromatische Verbindung abgetrennt und daß Iodidsalz in Jod zurückverwandelt wird.

2. Verfahren nach Anspruch 1, dadurch gekennzeichnet, daß der Substituent eine phenolische Hydroxylgruppe ist.

3. Verfahren nach Anspruch 1, dadurch gekennzeichnet, daß die aromatische Verbindung sowohl eine Aldehydgruppe als auch einen Hydroxy- oder Alkoxysubstituenten enthält.

4. Verfahren nach Anspruch 3, dadurch gekennzeichnet, daß als aromatische Verbindung Vanillin verwendet wird.

5. Verfahren nach einem der Ansprüche 1 bis 4, dadurch gekennzeichnet, daß die aromatische Verbindung mit Triiodidreagenz in Anwesenheit von Wasser umgesetzt wird, das 0,7 bis 1,25 Mol-Äquivalente Alkalihydroxid enthält.

6. Verfahren nach einem der Ansprüche 1 bis 4, dadurch gekennzeichnet, daß die aromatische Verbindung mit Triiodidreagenz in Gegenwart von 0,1 bis 20 mol% eines Säurekatalysators umgesetzt wird.

7. Verfahren nach einem der Ansprüche 1 bis 6, dadurch gekennzeichnet, daß als hydroxylierendes Mittel 0,5 bis 6 molares Hydroxid in Anwesenheit von 0,1 bis 20 mol% Kupfer oder Kupfer-I-Salz als Katalysator verwendet wird.

8. Verfahren nach einem der Ansprüche 1 bis 7, dadurch gekennzeichnet, daß nach der Abtrennung der hydroxyaromatischen Verbindung in Form einer organischen Phase durch Oxidation in Jod zurückverwandelt wird.

9. Verfahren nach Anspruch 8, dadurch gekennzeichnet, daß die Oxidation mit Chlor durchgeführt wird.

## Claims

1) Process for the hydroxylation of an aromatic compound with at least one electron donating nuclear substituent, wherein a iodide salt connected to the nucleus is exchanged by a hydroxyl group characterized in

- that the aromatic compound is caused to react in water with a triodiide reagent to a reaction mixture of iodoaromatic compound and iodide salt,

- that a hydroxylating agent is added to the reaction micture and the iodine atom of the iodoaromatic compound is exchanged by the hydroxyl group forming additional iodide salt, and

- that the hydroxy aromatic compound is separated and that the iodide salt is converted to iodine.

2) The process of claim 1, characterized in that the substituent is a phenolic hydroxyl group.

3) The process of claim 1, characterized in that the aromatic compound contain both an aldehyde group and a hydroxy- or alkoxy substituent.

4) The process of claim 3, characterized in that vanillin is used as aromatic compound.

5) The process of one of the claims 1 to 4, characterized in that the aromatic compound is reacted with triodiode reagent in the presence of water containing 0,7 to 1,25 molar equivalents of an alkali metal hydroxide.

6) The process of one of the claims 1 to 4, characterized in that the aromatic compound is reacted with triodide reagent in the presence of 0.1 to 20 molar equivalents of an acid catalysator.

7) The process of one of the claims 1 to 8, characterized in that in the presence of 0.1 to 20 mole% copper metal or cuprous salt acting as a catalysator 05. to 6 mole hydroxideas hydroxylating agent are used.

8) The process of one of the claims 1 to 7, characterized in that after separation of the hydroxy aromatic compound in form of an organic phase the iodidesalt is changed to iodine by oxidation.

9) The process of claim 8, characterized in that the oxidation is carried out with chlorine.

## Revendications

1. Procédé permettant d'hydroxyler un composé aromatique comportant, sur le noyau, au moins un substituant faisant fonction de donneur d'électron, dans lequel un atome d'iode lié au noyau est échangé contre un groupement hydroxyle, caractérisé en ce que le composé aromatique est mis a réagir dans l'eau avec un réactif triioduré pour obtenir un mélange réactionnel constitué d'un composé aromatique iodé et de sel ioduré, en ce qu'un agent hydroxylant est ajouté au mélange réactionnel, l'atome d'iode étant échangé contre le groupement hydroxyle en formant à nouveau du sel ioduré, et en ce que le composé aromatique hydroxylé est séparé, le sel ioduré étant retransformé en iode.

2. Procédé conforme à la revendication 1, caractérisé en ce que le substituant est un groupement hydroxyle phénolique.

3. Procédé conforme à la revendication 1, caractérisé en ce que le composé aromatique contient aussi bien un groupe aldéhyde qu'un substituant hydroxyle ou alkoxyle.

4. Procédé conforme à la revendication 3, caractérisé en ce que le composé aromatique employé est de la vanilline.

5. Procédé conforme à l'une quelconque des revendications 1 à 4, caractérisé en ce que le composé aromatique est mis à réagir avec un réactif triioduré en présence d'eau contenant de 0,7 à 1,25 équivalents molaires d'hydroxyde alcalin.

6. Procédé conforme à l'une quelconque des revendications 1 à 4, caractérisé en ce que le composé aromatique est mis à réagir avec un réactif triioduré en présence de 0,1 à 20 équivalents molaires de catalyseur acide.

7. Procédé conforme à l'une quelconque des revendications 1 à 6, caractérisé en ce que l'agent hydroxylant est un hydroxyde employé, à raison de 0,5 à 6 moles, en présence de 0,1 à 20 % en moles de cuivre ou de sel cuivreux utilisés comme catalyseur.

8. Procédé conforme à l'une quelconque des revendications 1 à 7, caractérisé en ce qu'après séparation du composé aromatique hydroxylé se présentant sous la forme d'une phase organique, le sel ioduré est retransformé en iode par oxydation.

9. Procédé conforme à la revendication 8, caractérisé en ce que l'oxydation est réalisée avec du chlore.